# EUROPEAN PATENT SPECIFICATION

(11) **EP 0 526 642 B1**
(45) Date of publication and mention of the grant of the patent: **01.04.1998**
(21) Application number: 92900597.3
(22) Date of filing: 13.12.1991
(51) Int. Cl.: C07J 63/00, A61K 31/705, A61K 35/78

(54) **TRITERPENE DERIVATIVE**
TRITERPENDERIVAT
DERIVE DE TRITERPENE

(30) Priority: 29.01.1991 JP 29372/91
(43) Date of publication of application: 10.02.1993
(73) Proprietor: SHIONOGI SEIYAKU KABUSHIKI KAISHA, Osaka 541 (JP)
(72) Inventor: FUJIMOTO, Masafumi, Ikoma-shi Nara 630-01 (JP); SAKURAI, Kensuke, Kitakaturagi-gun Nara 636 (JP); MIHARA, Shinichi, Yamatokooriyama-shi Nara 639-11 (JP); NAKAMURA, Miharu, Chuo-ku Kobe-shi Hyogo 650 (JP); KONOIKE, Toshiro, Suita-shi Osaka 564 (JP)
(74) Representative: VOSSIUS & PARTNER
(86) International application number: PCT/JP91/01707
(87) International publication number: WO 92/12991

(56) References cited:
- EP-A- 0 368 670
- JP-A-59 231 100
- JP-A-63 222 119
- CHEMICAL ABSTRACTS, vol. 82, no. 21, 26 May 1975, Columbus, Ohio, US; abstract no. 140328f, J. M. KOEKEMOER ET AL 'Chemistry of Melianthus comosus. VI. Structure of a new triterpenoid acid from the root bark.' page 648 ;column 1 ; & J. S. AFR. CHEM. INST. vol. 27, no. 3 , 1 March 1974 pages 131 - 136
- CHEMICAL ABSTRACTS, vol. 116, no. 13, 30 March 1992, Columbus, Ohio, US; abstract no. 124874w, S. SADDIQUI ET AL 'Isolation and structure elucidation of obtusilinin, a new terpenoid and 27-p-Z-coumaroyloxyursolic acid from the leaves of Plumeria obtusa.' page 483 ;column 2 ; & J. CHEM. SOC. PAK. vol. 13, no. 2 , 1 April 1991 pages 115 - 119

## Description

The present invention relates to useful compounds in the field of pharmaceuticals and the use thereof, in more detail, to endothelin-receptor antagonists comprising triterpene derivatives which are useful for preventing or treating diseases caused by excessive secretion of endothelin, and to novel triterpene derivatives.

Endothelin, which has been disclosed by M. Yanagisawa et al. (Nature, 332, P411, 1988), is an endothelium-derived 21-residue vasoconstrictor peptide . Excessive secretion of endothelin may be involved in various diseases, such as hypertension, coronary ischemia, encephalopathy, nephropathy, circulation failures of various organs, and asthma. In Kokai 2-273625, TXA₂-receptor antagonist and inhibitors of TXA₂-synthetase etc. are disclosed as substances capable of inhibiting the increase in intracellular calcium ion concentration, which is caused by excessive secretion of endothelin. However, nobody has reported on a substance capable of specifically inhibiting actions caused by endothelin. The development of such a new compound is desirable.

Some compounds similar to the present compounds in chemical structure are already known, however, there has been no report on the activity of those compounds as endothelin antagonist. For example, though 27-(3,4-dihydroxycinnamoyl-oxy)-3β-hydroxy-oleanolic acid which is analogous to the compounds of the present invention has been reported by J. M. Koekemore et al. in JOURNAL OF THE SOUTH AFRICAN CHEMICAL INSTITUTE (27, P131, 1974), there is no disclosure of any endothelin antagonist activity.

The isolation and structure of triterpenoids from the leaves of plumeria obtusa is disclosed in J. Chem. Soc. Pak. 13/2, 115 (1991) by S. Siddiqui et al.

This invention concerns active substances capable of specifically inhibiting the binding of endothelin to endothelin-receptors. The present invention provides pharmaceutical compositions useful as endothelin-receptor antagonists comprising triterpene derivatives represented by the formula (II): 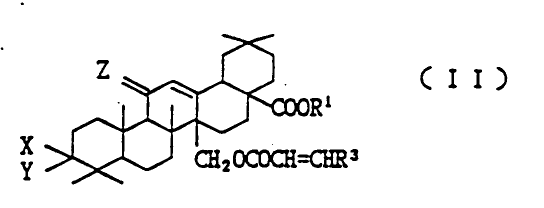 wherein, R¹ is hydrogen or a metabolizable ester-residue; R³ is an optionally substituted aryl or an optionally substituted aromatic heterocycle; X is hydrogen and Y is hydroxy or X and Y may form together oxo; Z is an oxygen or two hydrogen atoms, or the salts thereof.

Triterpene derivatives represented by the formula (I): 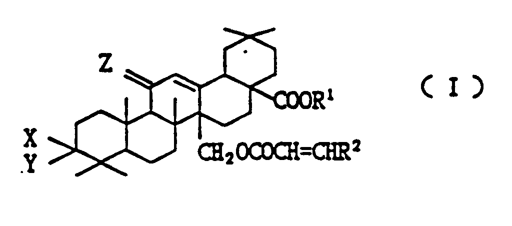 wherein, R¹ is hydrogen or a metabolizable ester-residue; R² is an optionally substituted aryl or an optionally substituted aromatic heterocycle; X is hydrogen and Y is hydroxy or X and Y may form together oxo, Z is an oxygen or two hydrogen atoms, provided that R² is not 3,4-dihydroxyphenyl when R¹ is hydrogen and Y is hydroxy and further provided that R² is not 4-hydroxyphenyl when R¹ is hydrogen, Y is hydroxy and Z is two hydrogen atoms, and the salts therof are novel compounds and the present invention provides also these compounds.

In the present specification, "metabolizable ester-residue" means an ester-residue which decomposes to reproduce carboxylic acids in the living body. "Aryl" means phenyl or (α- or β-) naphthyl. "Aromatic heterocycle" means aromatic 5- or 6- membered ring which contains one or more atoms selected from oxygen, sulfur, and/or nitrogen and may be condensed with a hydrocarbon or another heterocycle, and N- or S-oxide of the aromatic heterocycle is included in this definition. The substituent meant in the phrase "optionally substituted" includes, for example, halogen (F, Cl, Br, I), hydroxy, amino, amino substituted by lower alkanoyl, amino substituted by mono- or di- lower alkyl, carboxy, cyano, nitro, lower alkyl, lower alkoxy, and lower alkanoyloxy. In the case where Y is hydroxy, the hydroxy may be in α -or β-form.

Furthermore, in the case where R¹ is hydrogen, the present compounds may form salts with alkali metals (sodium, potasium etc) alkaline earth metals (calcium, magnesium etc) ammonia or an organic base (triethylammonium etc.).

The triterpene derivatives of the present invention can be extracted from Myrica cerifera L. by the following method for example. Namely, branches of the plant are subjected to extraction, at room temperature for a number of days, with a polar solvent including, e.g. alcohol such as methanol, ethanol, isopropanol, n-butanol, sec-butanol, and tert-butanol, acetone, and acetonitrile etc., then to extraction with an organic solvent hardly miscible with water, which includes chlorinated hydrocarbons such as chloroform, dichloromethane, ethyl acetate, and n-butanol etc. The eluate is separated by silica gel chromatography to obtain some of the compounds of the present invention. Furthermore, the thus-obtained compounds may be used to prepare other compounds of the invention having various other substituents as R².

The present compounds are useful for preventing and treating cardiovascular diseases such as vasoconstriction, hypertension, or the like because of their endothelin-receptor antagonist activity.

According to usual methods, the present compounds can be formulated, together with acceptable carriers, diluents, excipients etc., into compositions for administration via injection, oral and anal administration, or the like.

Though the dosage is not generally regulated because it changes depending on the objective effects of treatment, the administration method, or the age or weight of the patient, the daily dosage is usually in the range from 10mg to 2g, preferably from 50mg to 1g, for an adult.

The present invention is further explained by the following Examples and Experiments, which are not intended to limit the scope of the present invention at all.

### Example 1

### Preparation of Compounds 1 and 2

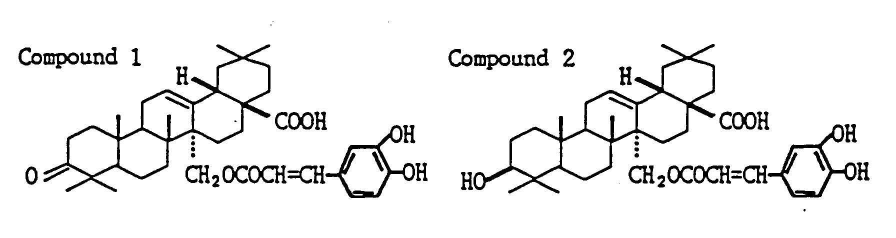

One kg of twigs of Myrica cerifera was pulverized, and soaked in 2L of methanol, then allowed to stand at room temperature for 6 days. The mixture was filtered then the filtrate was evaporated under reduced pressure. This process was repeated 3 times to obtain 75.4g of a course residue, 71g of which was distributed into chloroform (500ml×3) and water (1L) to obtain 10g of extract soluble in chloroform. The extract was subjected to chromatography with 130g of silica gel (230 to 400 mesh) containing 10% water, and eluted with chloroform and methanol mixed with chloroform. The portion eluated with a mixed solution of chloroform and methanol (98:2) was again subjected to the same chromatography, then the eluate (318mg) was subjected to high performance liquid chromatography with the column: DEVELOSIL ODS-10/20 (20mm × 250mm). The moving layer was a mixed solution of methanol and water (9:1) at a flow rate of 3ml/min.. An ordinary differential refractometer was used for the detection of the objective compounds. The retention time was 28 min. The eluated compounds were purified by repeating the method 3 to 5 times, whereby the objective esters were obtained.

### (Compound 1)

Yield: 103mg, Molecular Formula: C₃₉H₅₂O₇ (m/z 632), mp.177-182°C [α]_{D}+149.4±2.2° (25°C, c 0.88, MeOH)
¹H-NMR δ ppm (CDCl₃): 7.49(1H, d, J=15.8Hz), 7.05(1H, d, J=1.5Hz), 6.92(1H, dd, J=1.5, 8.3Hz), 6.85(1H, d, J=8.3Hz), 6.16(1H, d, J=15.8Hz), 5.64(1H, t ,J=3.2Hz), 4.36(1H, d, J=12.7Hz), 4.16(1H, d, J=12.7Hz), 2.93(1H, dd, J=3.8, 13.6Hz), 2.51-2.35(2H, m), 1.08, 1.04(×2), 0.93, 0.86, 0.84(3H each, s).
¹³C-NMR δ ppm (CDCl₃): 219.3, 181.3, 167.8, 147.8, 145.6, 145.3, 137.7, 126.9, 126.8, 122.1, 115.5, 115.0, 114.2, 65.6, 55.0, 47.9, 47.5, 46.4, 45.6, 44.9, 41.3, 40.0, 39.1, 37.0, 34.1, 33.6, 33.0, 32.7, 32.5, 30.7, 26.7, 24.1, 23.6, 23.6, 22.9, 21.5, 19.7, 17.9, 15.3.

### (Compound 2)

Amorphous, Molecular Formula: C₃₉H₅₄O₇, MS (SIMS Method) m/e 657[M+Na]⁺, [α]_{D}+170.4±1.6° (22°C, c=1.30, MeOH)
¹H-NMR δ ppm (CD₃OD): 7.50(1H, d, J=16.2Hz), 7.01(1H, d, J=1.6Hz), 6.91(1H, dd, J=8.2, 1.6Hz), 6.79(1H, d, J=8.2Hz), 6.17(1H, d, J=16.2Hz), 5.61(1H, m), 4.41(1H, d, J=12.4Hz), 4.14(1H, d,J=12.4Hz), 3.11(1H, m), 2.94(1H, br.d, J=11.0Hz), 0.77, 0.83, 0.94, 0.96(×3) (3H each, s).
¹³CNMR δ ppm (CD₃OD): 181.7, 168.8, 149.7, 146.9, 146.9, 138.9, 128.1, 127.5, 122.8, 116.6, 115.3, 115.0, 79.5, 66.7, 56.6, 50.0, 47.3, 46.6, 46.2, 42.5, 41.2, 39.8, 39.7, 38.3, 34.7, 34.4, 33.7, 33.5, 31.5, 28.7, 27.8, 25.1, 24.7, 24.1, 23.9, 19.5, 18.9, 16.3, 16.1.

### Reference Example 1

### Preparation of Compound A

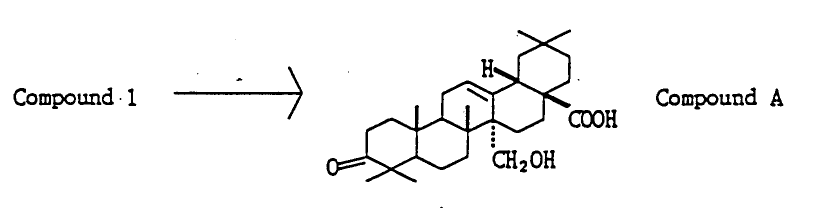

Compound 1 (110.0mg) was dissolved in 4ml of a solution of 3% KOH dissolved in methanol, and the mixture was heated under reflux for 3 hours. Water was added thereto, from which methanol was evaporated under reduced pressure. The resulting solution was acidified with 1N sulfuric acid, extracted with ethyl acetate (15ml × 3), which was washed with water and evaporated. The obtained residue was subjected to silica gel chromatography to obtain 14.6mg of compound A from the portion of 2% methanol/chloroform. Mp. 226-227°C, [α]_{D}+91.3° (c=1.0, CHCl₃) m/e 470 (C₃₀H₄₆O₄)

A wide variety of compounds substituted at the 27-position are prepared by applying the following methods.
(a) The direct acylation for compound A
(b) Horner-Emmons method for Compound A

### (a) The Direct Acylation

Compound A is treated with acid halides (R²CH=CHCO-halogen) or reactive derivatives of carboxylic acids such as [(R²CH=CHCO)₂O)] in the presence of dimethylaminopyridine (DMAP), to give the present compounds represented by the formula (II).

### (b) Horner-Emmons Method

Dimethylphosphonoacetic anhydride, which was prepared from, dimethylphosphonoacetic acid and dicyclohexylcarbodiimide (DCC), is reacted with compound A in the presence of DMAP to give the dimethyl phosphonoacetate of compound A (hereinafter referred to as compound B). Compound B was condensed with aldehydes (R²CHO) in the presence of either cesium carbonate or lithium chloride - triethylamine, to give Compound (I).

### Example 2

### Preparation of Compound 3

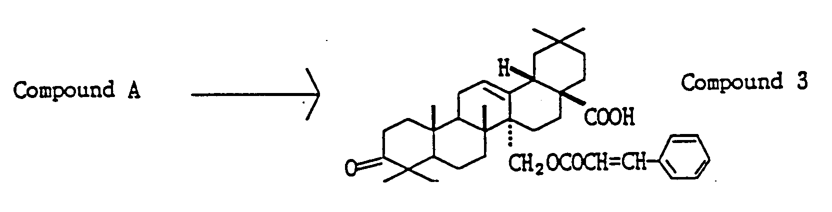

To a solution of 13.2mg of compound A (0.028mmole) dissolved in 0.6ml of dichloromethane, 10.3mg of dimethylaminopyridine (DMAP, 0.0843mg, 3eq.) were added, and then 11.7mg of cinnamoyl chloride (0.07mmole, 12.5eq.). The mixture was allowed to stand at room temperature over night, then condensed to give a solid residue. The residue was dissolved in 2ml of THF and, under ice-cooling, 1ml of an aqueous solution of 0.1N sodium hydroxide was added to the resulting mixture and the mixture was stirred for 30 min. Hydrochloric acid was added to acidify the reaction mixture, then the resulting mixture was extracted with ethyl acetate. The organic layer was washed with water, dried over magnesium sulfate, and subjected to silica gel chromatography with hexane-ethyl acetate (2:1) as eluent, to give 15.2mg of the objective compound 3 in 90% yield. Rf=0.6 (CHCl₃:MeOH=10:1)
¹HNMR δ (ppm) (CDCl₃):0.85(s,6H), 0.92(s,3H), 1.03(s,3H), 1.04(s,3H), 1.08(s,3H), 1.0-2.1(m,20H), 2.2-2.6(m,2H), 2.8-3.0(m,1H), 4.15,4.38 (ABq,2H,J=13.4Hz), 5.67(br.s,1H), 6.37(br.s,1H), 7.4-7.6(m,5H), 7.62 (d,1H, J=15.8Hz)

### Example 3

### Preparation of Compound 4

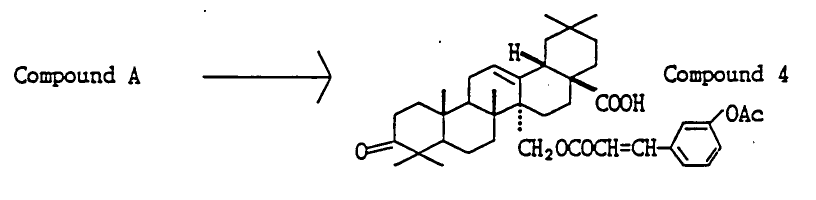

Compound A (5mg, 0.0106mmole), 5.2mg of DMAP (4eq.), and 13mg of 3-acetoxycinnamic anhydride (3eq.) were dissolved in 0.14ml of dichloromethane, and the mixture was allowed to stand at room temperature for 3 hours. The reaction mixture was purified by using silica gel chromatography with CHCl₃-MeOH as eluent, to give 4.6mg of compound 4 in 66% yield. Rf=0.8 (CHCl₃ :MeOH=10:1)
¹H-NMR δ ppm (CDCl₃): 0.83(s,3H), 0.85(s,3H), 0.93(s,3H), 1.02(s,3H), 1.04(s,3H), 1.08(s,3H), 1.1-2.1(m,20H), 2.34(s,3H), 2.3-2.6(m,2H), 2.8-3.0(m,1H), 4.16,4.34(ABq,2H, J=12.8Hz), 5.65(br.s,1H), 6.34(d,1H,J= 16.2Hz), 7.1-7.2(m,1H), 7.23(br.s,2H), 7.3-7.5(m,2H), 7.59(d,1H,J= 16.2Hz)

### Example 4

### Preparation of Compound 5

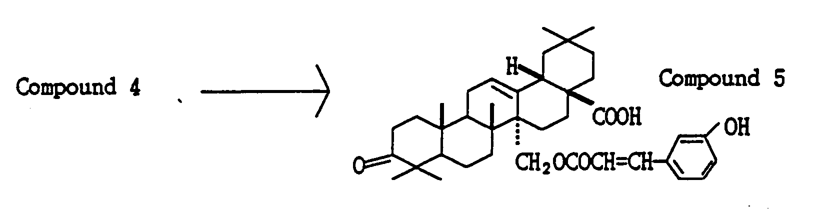

To a solution of 9.9 mg of compound 4 dissolved in 1ml of methanol, a solution of 3% KOH dissolved in methanol was added under ice-cooling. The mixture was stirred for 30 min. and poured into dil. aqueous hydrochloric acid, then extracted with ethyl acetate. The organic layer was dried, condensed, and subjected to silica gel chromatography with CHCl₃-MeOH as eluent, to give compound 5. Rf=0.6 (CHCl₃:MeOH=10:1)
¹H-NMR δ ppm (CDCl₃): 0.83(s,3H), 0.86(s,3H), 0.93(s,3H), 1.03(s,3H), 1.05(s,3H), 1.08(s,3H), 1.1-2.1(m,20H), 2.3-2.5(m,2H), 2.8-3.0(m,1H), 4.19,4.36(ABq,2H,J=13.0Hz), 5.65(br.s,1H), 6.32(d,1H,J=7.9, 2.4, 0.8Hz), 6.90(ddd,1H,J=7.9,2.4,0.8Hz), 6.97(t,1H,J=1.8Hz), 7.07(d,1H,J=7.9Hz), 7.27(t,1H,J=7.9Hz), 7.57(d,1H,J=15.8Hz)

### Example 5

### Preparation of Compound 6

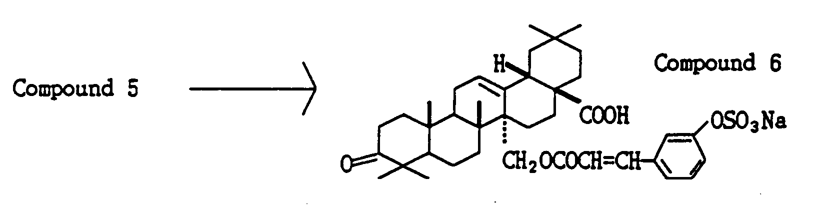

To a solution of 3.1mg of compound 5 (0.005mmole) dissolved in 50 µl of DMF, 8 µl of 1H-NaOH was added, followed by 1mg of sulfur trioxide-trimethylamine complex. After repeating the process 10 times, the resulting mixture was evaporated under reduced pressure and dried to give a solid residue. To the residue, 1ml of water and 1N-NaOH were added, then the mixture was subjected to the chromatography using Diaion HP20® (water/MeOH:50/50), to give 1.4mg of compound 6 in 38% yield. Rf=0.4 (ethyl acetate: acetic acid:water=30:1:1)

### Example 6

### Preparation of Compound 7

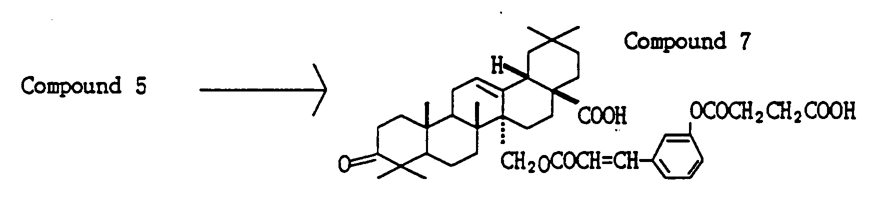

To a solution of 7mg of compound 5 (0.0011mmole) dissolved in 0.1ml of dichloromethane, 11mg of DMAP (8eq.) and 6.6mg of succinic anhydride (6eq.) were added and the mixture was stirred at room temperature for 5 hours. The reaction mixture was poured into dil. hydrochloric acid and extracted with ethyl acetate. The extracts were dried, evaporated, and subjected to silica gel chromatography (CHCl₃:MeOH=10:1), to give 3.1mg of compound 7 in 39% yield. Rf=0.4 (CHCl₃:MeOH=10:1)
¹H-NMR δ ppm (CDCl₃): 0.83(s,3H), 0.86(s,3H), 0.93(s,3H), 1.04(s,3H), 1.05(s,3H), 1.08(s,3H), 1.0-2.1(m,20H), 2.2-2.8(m,2H), 2.8-3.0(m,3H), 4.2-4.4(m,2H), 5.63(br.s,1H), 6.32(d,1H,J=16.0Hz), 7.1-7.4(m,4H), 7.58 (d,1H,J=16.0Hz)

### Example 7

### Preparation of Compound 8

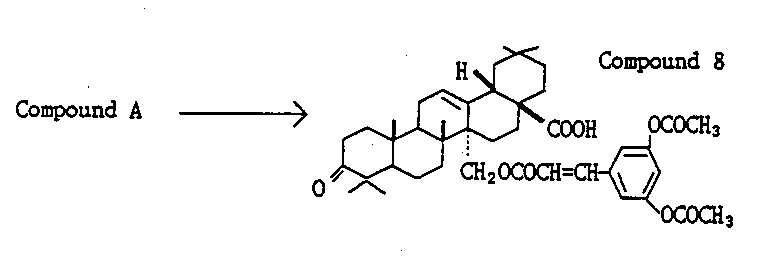

To a solution of 5.0mg of compound A (0.011mmole) dissolved into 0.15ml of dichloromethane, 5.2mg of DMAP (4eq.) was added, followed by 10mg of 3,5-diacetoxycinnamoyl chloride (3eq.). The mixture was allowed to stand at room temperature for 2 hours and the reaction solution was evaporated and dried to obtain a solid, which was dissolved into 1ml of THF. Under ice-cooling, 1ml of an aqueous solution of 0.1N sodium hydroxide was added thereto and the resulting mixture was stirred for 30 min. The reaction solution was acidified with 0.1N HCl and subjected to extraction with ethyl acetate. The organic layer was washed with water, dried over anhydrous magnesium sulfate, evaporated, and subjected to silica gel chromatography, to give 1.9mg of compound 8 in 25% yield.
¹H-NMR δ ppm (CDCl₃):0.82(s,3H), 0.86(s,3H), 0.93(s,3H), 1.04(s,6H), 1. 09(s,3H), 1.0-2.1(m,20H), 2.2-2.8(m,2H), 2.31(s,6H), 2.8-3.0(m,1H), 4.15, 4.38(ABq,2H,J=16.0Hz), 5.67(br.s,1H), 6.29(d,1H,J=16.0Hz), 6.66 (t,1H,J=2.0Hz), 6.81(s,2H), 7.54(d,1H,J=16.0Hz)

### Example 8

### Preparation of Compound 9

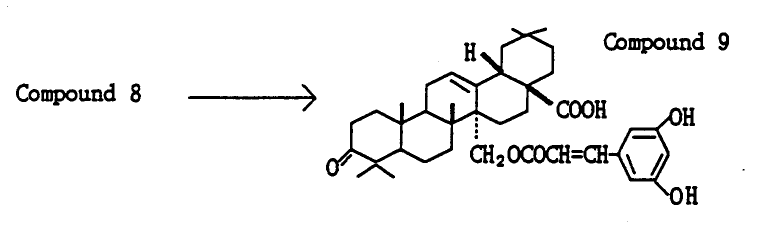

To a solution of 1.9mg of compound 8 (0.003mmole) dissolved in 0.5ml of methanol, 0.1ml of a solution of 3% KOH dissolved in methanol was added under ice-cooling and the resulting mixture was stirred for 30 min. The reaction solution was acidified with 1N HCl, then extracted with ethyl acetate. The organic layer was washed with water, dried over anhydrous magnesium sulfate, evaporated, and subjected to silica gel chromatography, to give 0.4mg of compound 9 in 24% yield.
¹HNMR δ ppm (CD₃OD):0.85(s,3H), 0.90(s,3H), 0.94(s,3H), 1.04(s,3H), 1.07 (s,6H), 1.1-2.1(m,20H), 2.2-2.7(m,2H), 2.9-3.1(m,1H), 4.17,4.18 (ABq, 2H, J=13.0Hz), 5.63(br.s,1H), 6.27(d,1H, J=16.0Hz), 6.30(t,1H,J=2.0Hz), 6.46(d,2H,J=2.2Hz), 7.54(d,1H,J=16.0Hz)

### Reference Example 2

### Preparation of dimethoxyphosphonoacetic anhydride

To a solution of 1.68g of dimethoxyphosphonoacetic acid (10mmole) dissolved in THF, 980mg of DCC (4.75mmole) was added. The mixture was stirred at room temperature for 8 hours and 20ml of ether was added thereto . Then the resulting solution was allowed to stand at -20°C. The precipitate was removed by filtration, whereby a solution of dimethoxyphosphonoacetic anhydride was obtained (calcd. yield: 0.11 mole/l).

### Reference Example 3

### Preparation of Compound B

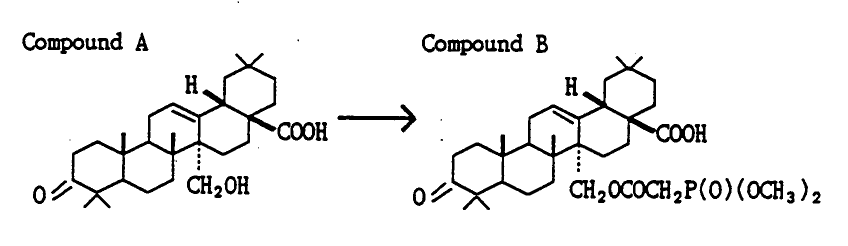

To a mixture of 15.6mg of compound A (0.033mmole) with 16mg of DMAP (4eq.), 1.2ml of the above solution of dimethoxyphosphonoacetic anhydride (4eq.) was added. Dichloromethane (0.3ml) was added thereto and the resulting mixture was stirred at room temperature for 5 hours, then subjected to extraction with ethyl acetate. The extracts were evaporated, and purified by using silica gel chromatography (CHCl₃:MeOH=20:1), to give 13.3mg of compound B in 65% yield.
¹HNMR δ ppm (CDCl₃): 0.81(s,3H), 0.89(s,3H), 0.94(s,3H), 1.03(s,3H), 1.04(s,3H), 1.09(s,3H), 1.1-2.1(m,20H), 2.2-2.7(m,2H), 2.8-3.0(m,1H), 2.95(d,2H,J=21.8Hz), 3.81(d,6H,J=11.2Hz), 4.15,4.33(ABq,2H,J=12.8Hz), 5.61(br.s,1H)

### Example 9

### Preparation of Compounds 10 and 11

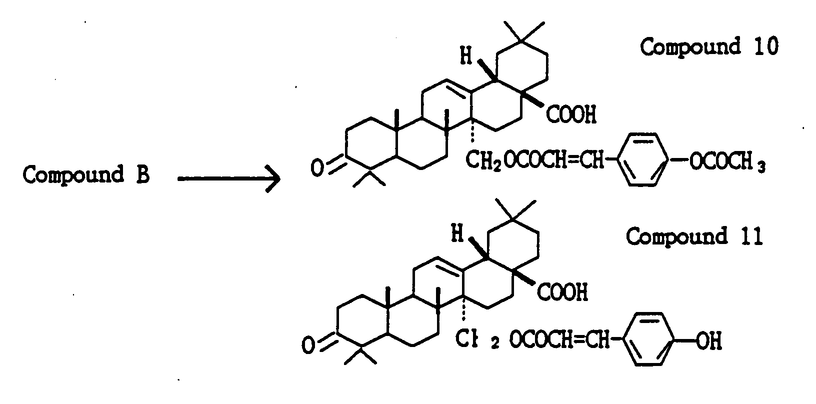

To a solution of 2.76mg of compound B dissolved in 0.1ml of 2-propanol, 2.5µl of 4-acetoxybenzaldehyde (4eq.) was added, followed by 3mg of cesium carbonate, and the mixture was stirred at room temperature for 30min. The reaction mixture was poured into dil. hydrochloric acid and extracted with ethyl acetate. The organic layer was evaporated and subjected to silica gel chromatography (CHCl₃:MeOH=100:1-20:1), to give 1.1mg of compound 10 [Rf=0.8 (CHCl₃: MeOH=10:1)] and 1.0mg of compound 11 [Rf=0.6 (CHCl₃:MeOH=10:1)] were obtained.

### (Compound 10)

¹HNMR δ ppm (CDCl₃):0.84(s,3H), 0.85(s,3H), 0.93(s,3H), 1.02(s,3H), 1.04 (s,3H), 1.08(s,3H), 1.0-2.1(m,20H), 2.32(s,3H), 2.3-2.6(m ,2H), 2.8-3.0 (m,1H), 4.15,4.38(ABq,2H,J=12.6Hz), 5.66(br.s,1H), 6.3(d,1H,J=15,8Hz), 7.13(d,1H,J=8.6Hz), 7.54(d,2H,J=8.6Hz), 7.60(d,1H,J=15.8Hz)

### (Compound 11)

¹H-NMR δ ppm (CDCl₃):0.84(s,3H), 0.85(s,3H), 0.93(s,3H), 1.03(s,3H), 1.04(s,3H), 1.07(s,3H), 1.0-2.1(m,20H), 2.2-2.6(m,2H), 2.8-3.0(m,1H), 4.13,4.37(ABq,2H,J=12.6Hz), 5.65(br.s,1H), 6.22(d,1H,J=15.8Hz), 6.84(d, 2H,J=8.4), 7.41(d,2H,J=8.4Hz), 7.56(d,1H,J=15.8Hz)

### Example 10

### Preparation of Compound 12

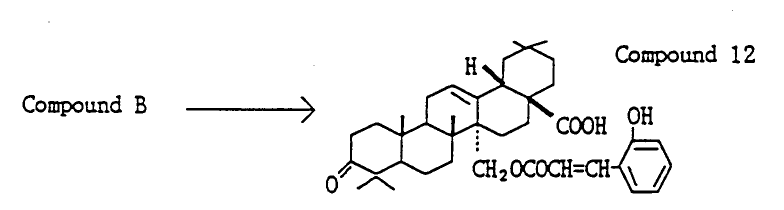

To a solution of 2.76mg of compound B and 1.4 µl of 2-hydroxybenzaldehyde (3eq.) dissolved in 0.1ml of isopropanol, 3mg of cesium carbonate was added. The mixture was stirred at room temperature for 30min. and subjected to extraction with ethyl acetate. The organic layer was washed with water, dried, and subjected to silica gel chromatography (CHCl₃:MeOH=100:1-20:1), to give 1.7mg of compound 12 was obtained. Rf=0.5 (CHCl₃:MeOH=10:1)
¹H-NMR δ ppm (CDCl₃):0.84(s,3H), 0.85(s,3H), 0.92(s,3H), 1.03(s,6H), 1.0 8(s,3H), 1.1-2.2(m,20H), 2.2-2.6(n,2H), 2.8-3.0(m,1H), 4.19,4.36(ABq,2H, J=12.8Hz), 5.66(br,s,1H), 6.46(d,1H,J=14.0Hz), 6.63(dd,1H,J=8.0, 1.0Hz), 6.95(t,1H,J=8.0), 7.2-7.3(m,1H), 7.49(dd,1H,J=8.0, 1.0), 7.92(d,1H,J=14. 0Hz)

### Example 11

### Preparation of Compound 13

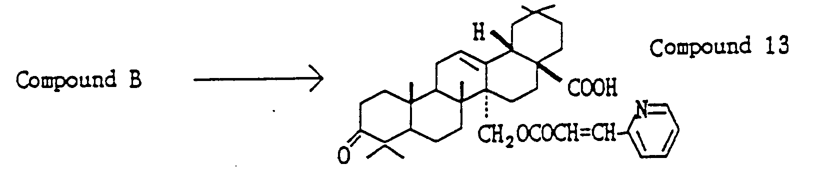

To a solution of 33.7mg of compound B dissolved in 0.9ml of acetonitrile, 17 µl of triethylamine (2.2eq.), 5.7 µl of 2-pyridinecarboxaldehyde (1.1eq.) and 5.2mg of lithium bromide (1.1eq.) were added, then the mixture was stirred at room temperature for 1 hour. The reaction mixture was evaporated and subjected to silica gel chromatography (CHCl₃:MeOH=50:1), to give 14.6 mg of compound 13, at a yield of 75%. Rf=0.6 (CHCl₃:MeOH= 10:1)
¹H-NMR δ ppm (CDCl₃):0.83(s,3H), 0.86(s,3H), 0.93(s,3H), 1.01(s,3H), 1.03(s,3H), 1.07(s,3H), 1.1-2.1(m,20H), 2.2-2.6(m,2H), 2.8-3.0(m,1H), 4.19, 4.40(ABq,2H,J=13.0Hz), 5.66(br.s,1H), 6.86(d,1H, J=15.6HZ), 7.2-7.4 (m,1H), 7.44(d,1H,J=5.6Hz), 7.64(d,1H,J=15.6Hz), 7.76(td,J=7.6, 2.0Hz), 8.2-8.3(m,1H)

### Example 12

### Preparation of Compound 14

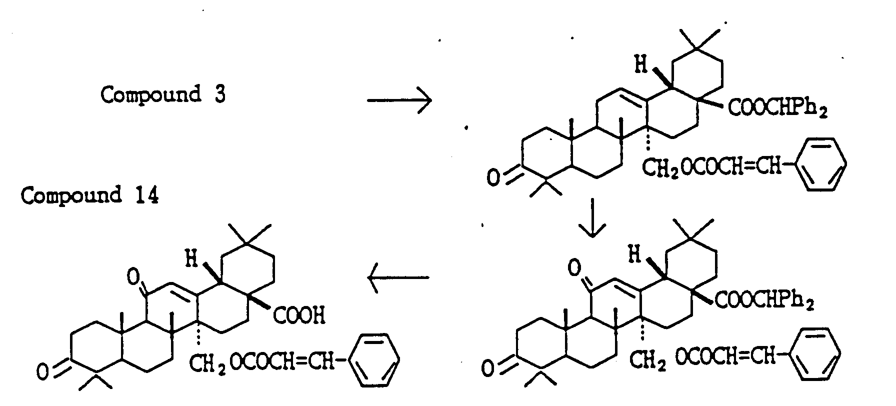

To a solution of 4.0mg of compound 3 (0.007mmole) dissolved in 8 µl of dichloromethane, 5.2mg of diphenyldiazomethane (4eq.) was added and the mixture was stirred at room temperature over night. A drop of acetic acid was added thereto and the resulting solution was stirred for 30 min., then evaporated and dried to obtain a solid, which was separated and purified with silica gel chromatography (hexane: ethyl acetate=4:1), to give 5mg of the diphenylmethyl ester of compound 3. To a solution of 5mg of the ester (0.007mmole) dissolved in 15 µl of benzene, a catylitic pulverized Molecular Sieve (4A) and 36mg of pyridiniumchloroformate (PCC) (4eq.) were added, and the mixture was heated under reflux for 24 hours. The resulting solution was evaporated and dried to obtain a solid, which was separated and purified by using silica gel chromatography (hexane: ethyl acetate=4:1), to give the diphenylmethyl ester of compound 14.

To a solution of the above ester of compound 14 dissolved in 100 µl of dichloromethane, 20 µl of anisole and 80 µl of trifluoroacetic acid were added, then the mixture was stirred for 30min. The resulting solution was evaporated to dryness to give a solid residue, which was separated and purified by using silica gel chromatography (CHCl₃/MeOH), to give 0.1mg of compound 14.
¹H-NMR δ ppm (CDCl₃): 0.86(s,3H), 0.96(s,3H), 1.05(s,3H), 1.06(s,3H), 1. 09(s,3H), 1.20(s,3H), 1.1-2.1(m,20H), 2.2-2.6(m,2H), 2.8-3.0(m,1H), 4.45 4.5(ABq,2H,J=13.0Hz), 5.99(s,1H), 6.34(d,1H,J=16.0Hz), 7.1-7.2(m,1H), 7.23(br.s,1H), 7.3-7.5(m,2H), 7.59(d,1H,J=16.0Hz)

It has been suggested that endothelin is related to cardiovascular failure, because it shows a wide variety of pharmacological effects, e.g., smooth muscle constriction, by activating its specific receptors existing on cell membranes at various organs such as blood vessels or trachea. The following Experimental Examples show the anti-endothelin activity of the present compounds.

### Experimental Example 1

### Inhibitory Effect on the Binding of ¹²⁵I-Endothelin-1 to the Receptor

### (Method)

Membrane fractions were prepared from porcine and rat hearts, rat thoracic aorta, and the media of porcine aorta. Each membrane fraction was incubated, in the presence or absence of the present compound 1 with 25pM of ¹²⁵I-endothelin-1 at 37°C for 1 hour. After the reaction was completed, ¹²⁵I-endothelin-1 bound to each membrane fraction was separated by rapid filtration through glass fiber filters and its radioactivity was determined with a gamma-counter. The specific binding was calculated by subtracting, from the total binding, the non-specific binding which was determined in the presence of 10⁻⁷M of non-radioactive endothelin-1. The concentration of the present compound 1 to inhibit 50% of the specific binding (i.e., IC₅₀) is shown at Table 1.

**[Table 1]**

| Kind of Membrane Ingredient | IC₅₀ (nM) |
|---|---|
| the media from porcine aorta | 3.2×10⁻⁸ |
| porcine heart | 8.3×10⁻⁸ |
| rat aorta | 5.6×10⁻⁸ |

### Experimental Example 2

### Inhibition of the endothelin-1-induced increase in cytosolic calcium ion concentration

### (Method)

The suspension of rat aortic smooth muscle A7r5 cells, obtained from Dainippon Pharmaceutical Co., Ltd., loaded with 2µM of fura-2 (Dojin) was put into a cuvette, and then the change in fluorescence intensity was determined with a calcium analyzer (CAF100, Nippon Bunkou Co.). In the determination, the exciting was performed at 340nm and 380nm and the recording at 510nm. Calculation for the concentration of cytosolic calcium ion was performed according to the method of Grynkiewicz et al. (J.Biol.Chem., 260, P.3440-3450, 1985). The experiment was performed as follows: at first, the present compounds were added to the cell suspension in the cuvette and the mixture was incubated for 1 min. Then, 10⁻⁸M of endothelin-1 was added thereto and the change in fluorescence intensity was determined. The concentration of the present compounds to inhibit the endothelin-1-induced increase in cytosolic calcium ion by 50%, (i.e., IC₅₀) is shown at Table 2.

**[Table 2]**

| Compound No. | IC₅₀ (×10⁻⁸) |
|---|---|
| 1 | 1.1 |
| 3 | 1.8 |
| 4 | 3.4 |
| 5 | 1.1 |
| 6 | 1.4 |
| 7 | 0.9 |
| 8 | 26.0 |
| 9 | 0.45 |
| 10 | 18.0 |
| 11 | 2.7 |
| 12 | 2.7 |
| 13 | 12.5 |
| 14 | 1.0 |

At a concentration of 10⁻⁸M, the present compounds did not show any significant effects on the actions caused by bradykinin, bombesin, or TXA₂-receptor agonist.

As apparent from the above results, the present compounds bind to the endothelin-receptor and specifically inhibit actions caused by endothelin. Accordingly, the present compounds are expected to be effective in preventing or treating various diseases caused by excessive secretion of endothelin, for example, hypertension, coronary ischemia, encepholapathy, nephropathy, circulation failure of various oragans, and asthma.

## Claims (Claims for the following Contracting State(s): AT, BE, CH, DE, DK, FR, GB, IT, LI, LU, MC, NL, SE)

1. A triterpene of the following formula (I): 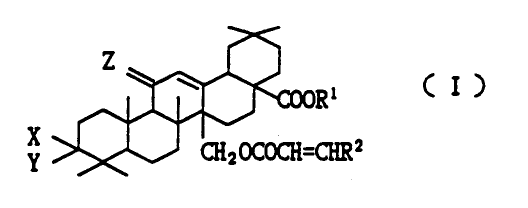 wherein, R¹ is hydrogen or a metabolizable ester-residue; R² is an optionally substituted aryl or an optionally substituted aromatic heterocycle; X is hydrogen and Y is hydroxy or X and Y may form together oxo; Z is an oxygen or two hydrogen atoms, provided that R² is not 3,4-dihydroxyphenyl when R¹ is hydrogen and Y is hydroxy and further provided that R² is not 4-hydroxyphenyl when R¹ is hydrogen, Y is hydroxy and Z is two hydrogen atoms, or the salts thereof.

2. The compound claimed in Claim 1, wherein R¹ is hydrogen; X and Y form together oxo.

3. The compound claimed in Claim 1 or 2, wherein R² is phenyl, hydroxyphenyl, or dihydroxyphenyl.

4. A pharmaceutical composition containing a triterpene of the following formula (II): 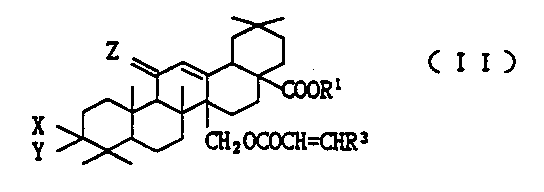 wherein R¹ is hydrogen or a metabolizable ester-residue; R³ is an optionally substituted aryl or an optionally substituted aromatic heterocycle; X is hydrogen and Y is hydroxy or X and Y may form together ozo; Z is an oxygen or two hydrogen atoms, or the salts thereof.

5. The pharmaceutical composition claimed in Claim 4, wherein R¹ is hydrogen; X and Y form together oxo.

6. The pharmaceutical composition claimed in Claim 4 or 5, which has antagonistic activities to endothelin-receptors.

7. The pharmaceutical composition claimed in claim 4 or 5 for use in preventing or treating diseases caused by endothelin.

8. A triterpene of the following formula:

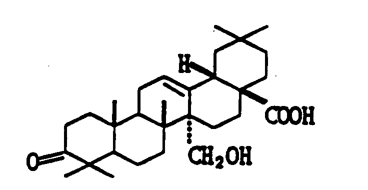

## Claims (Claims for the following Contracting State(s): ES)

1. A process for preparing a triterpene of the formula (I): 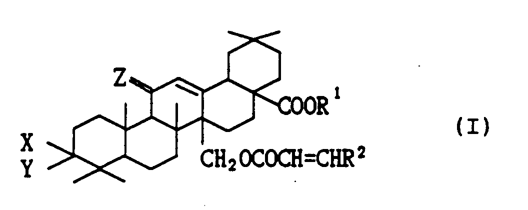 wherein R¹ is hydrogen or a metabolic ester-residue; R² is an optionally substituted aryl or an optionally substituted aromatic heterocycle; X is hydrogen and Y is hydroxy or X and Y may form together oxo; Z is an oxygen or two hydrogen atoms; provided that R² is not 3,4-dihydroxyphenyl when R¹ is hydrogen and Y is hydroxy, or the salts thereof, which comprises hydrolyzing a compound of the formula: 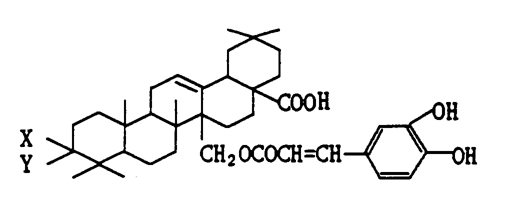 wherein X is hydrogen and Y is hydroxy or X and Y may form together oxo, to give the corresponding alcohol, which is subsequently subjected to:
(a) a reaction with an acid halide or an acid anhydride of the formulae:
R²CH=CHCO-halogen or (R²CH=CHCO)₂O)
wherein R² is the same as defined above, or
(b) a reaction with dimethylphosphonoacetic anhydride to give the corresponding dimethyl phosphonoacetate, which is reacted with a compound of the formula:
R²CHO
wherein R² is the same as defined above,
and, if necessary, further oxidation, followed by optional esterification/salt formation.

2. The method according to Claim 1, wherein R¹ is hydrogen; X and Y form together oxo.

3. The method according to Claim 1 or 2, wherein R² is phenyl, hydroxyphenyl, or dihydroxyphenyl.

4. A method for the preparation of a pharmaceutical composition containing a triterpene of the following formula (II): 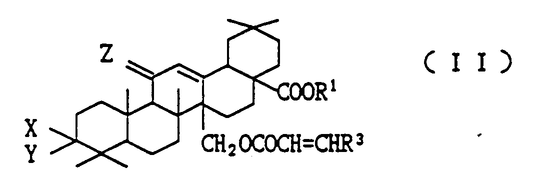 wherein R¹ is hydrogen or a metabolizable ester-residue; R³ is an optionally substituted aryl or an optionally substituted aromatic heterocycle; X is hydrogen and Y is hydroxy or X and Y may together form oxo; Z is an oxygen or two hydrogen atoms, or the salts thereof, comprising combining said triterpene with a pharmaceutically acceptable carrier.

5. The method according to claim 4, wherein R¹ is hydrogen; X and Y together form oxo.

6. The method according to claim 4 or 5 wherein the pharmaceutical composition prepared has antagonistic activities against endothelin-receptors.

7. The method according to claim 4 or 5 wherein the pharmaceutical composition prepared is useful in preventing or treating diseases caused by endothelin.

## Claims (Claims for the following Contracting State(s): GR)

1. A triterpene of the following formula (I): 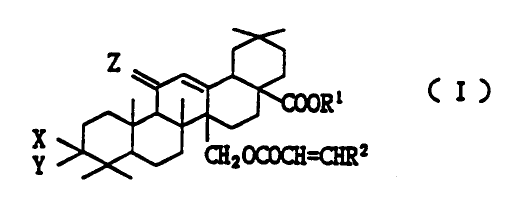 wherein, R¹ is hydrogen or a metabolizable ester-residue; R² is an optionally substituted aryl or an optionally substituted aromatic heterocycle; X is hydrogen and Y is hydroxy or X and Y may form together ozo; Z is an oxygen or two hydrogen atoms, provided that R² is not 3,4-dihydroxyphenyl when R¹ is hydrogen and Y is hydroxy and further provided that R² is not 4-hydroxyphenyl when R¹ is hydrogen, Y is hydroxy and Z is two hydrogen atoms, or the salts thereof.

2. The compound claimed in Claim 1, wherein R¹ in hydrogen; X and Y form together oxo.

3. The compound claimed in Claim 1 or 2, wherein R² is phenyl, hydroxyphenyl, or dihydroxyphenyl.

4. A method for the preparation of a pharmaceutical composition containing a triterpene of the following formula (II): 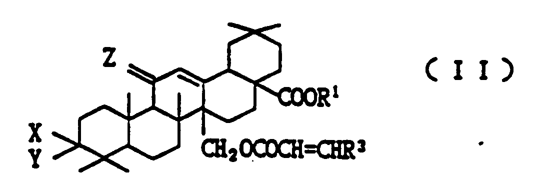 wherein R¹ is hydrogen or a metabolizable ester-residue; R³ is an optionally substituted aryl or an optionally substituted aromatic heterocycle; X is hydrogen and Y is hydroxy or X and Y may together form oxo; Z is an oxygen or two hydrogen atoms, or the salts thereof, comprising combining said triterpene with a pharmaceutically acceptable carrier.

5. The method according to claim 4, wherein R¹ is hydrogen; X and Y together form oxo.

6. The method according to claim 4 or 5 wherein the pharmaceutical composition prepared has antagonistic activities against endothelin-receptors.

7. The method according to claim 4 or 5 wherein the pharmaceutical composition prepared is useful in preventing or treating diseases caused by endothelin.

8. A triterpene of the following formula:

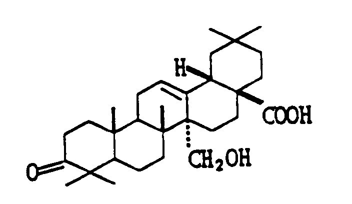

## Patentansprüche (Patentansprüche für folgende(n) Vertragsstaat(en): AT, BE, CH, DE, DK, FR, GB, IT, LI, LU, MC, NL, SE)

1. Triterpen der folgenden Formel (I) 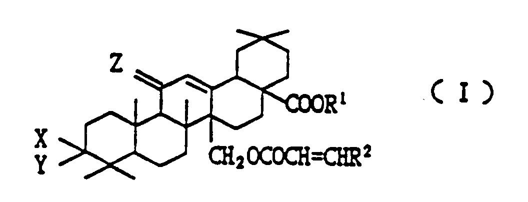 wobei R¹ ein Wasserstoffatom oder eine metabolisierbare Estergruppe ist; R² ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, mit der Maßgabe, daß R² keine 3,4-Dihydroxyphenylgruppe ist, wenn R¹ ein Wasserstoffatom ist und Y eine Hydroxylgruppe ist und weiterhin mit der Maßgabe, daß R² keine 4-Hydroxyphenylgruppe ist, wenn R¹ ein Wasserstoffatom ist, Y eine Hydroxylgruppe ist und Z zwei Wasserstoffatome bedeutet; oder die Salze davon.

2. Verbindung nach Anspruch 1, wobei R¹ ein Wasserstoffatom ist und X und Y zusammen eine Oxogruppe bilden.

3. Verbindung nach Anspruch 1 oder 2, wobei R² eine Phenyl-, Hydroxyphenyl- oder Dihydroxyphenylgruppe ist.

4. Arzneimittel, umfassend ein Triterpen der folgenden Formel (II) 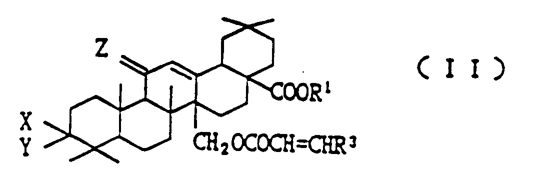 wobei R¹ ein Wasserstoffatom oder eine metabolisierbare Estergruppe ist; R³ ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, oder die Salze davon.

5. Arzneimittel nach Anspruch 4, wobei R¹ ein Wasserstoffatom ist und X und Y zusammen eine Oxogruppe bilden.

6. Arzneimittel nach Anspruch 4 oder 5, das antagonistische Wirkungen gegenüber Endothelin-Rezeptoren aufweist.

7. Arzneimittel nach Anspruch 4 oder 5 zur Verwendung bei der Prophylaxe oder Behandlung von Krankheiten, die durch Endothelin hervorgerufen werden.

8. Triterpen der folgenden Formel:

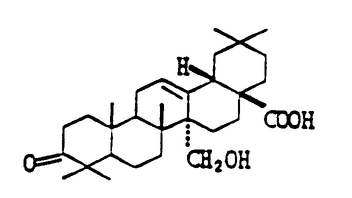

## Patentansprüche (Patentansprüche für folgende(n) Vertragsstaat(en): ES)

1. Verfahren zur Herstellung eines Triterpens der folgenden Formel (I) 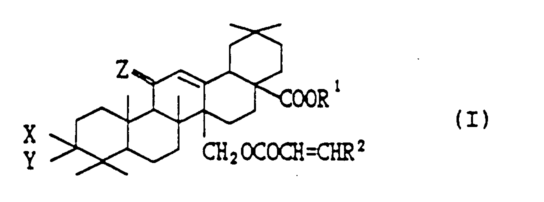 wobei R¹ ein Wasserstoffatom oder eine metabolisierbare Estergruppe ist; R² ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, mit der Maßgabe, daß R² keine 3,4-Dihydroxyphenylgruppe ist, wenn R¹ ein Wasserstoffatom ist und Y eine Hydroxylgruppe ist; oder der Salze davon, umfassend das Hydrolysieren einer Verbindung der Formel 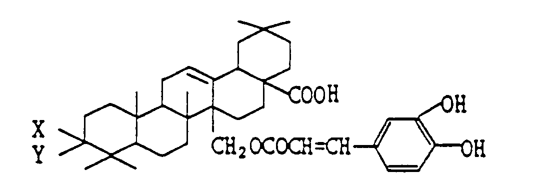 wobei X ein Wasserstoffatom ist und Y eine Hydroxylgruppe ist oder X und Y zusammen eine Oxogruppe bilden, wodurch der entsprechende Alkohol erhalten wird, der anschließend
a) mit einem Säurehalogenid oder einem Säureanhydrid der Formeln
R²CH=CHCO-Halogen oder (R²CH=CHCO)₂O),
wobei R² wie vorstehend definiert ist, umgesetzt wird oder
b) mit Dimethylphosphonessigsäureanhydrid umgesetzt wird, wodurch das entsprechende Dimethylphosphonacetat erhalten wird, das mit einer Verbindung der Formel
R²CHO umgesetzt wird,
wobei R² wie vorstehend definiert ist,
und, falls es notwendig ist, gefolgt von einer Oxidation, gegebenenfalls gefolgt von einer Veresterung/Salz-Bildung.

2. Verfahren nach Anspruch 1, wobei R¹ ein Wasserstoffatom ist und X und Y zusammen eine Oxogruppe bilden.

3. Verfahren nach Anspruch 1 oder 2, wobei R² eine Phenyl-, Hydroxyphenyl- oder Dihydroxyphenylgruppe ist.

4. Verfahren zur Herstellung eines Arzneimittels, umfassend ein Triterpen der folgenden Formel (II) 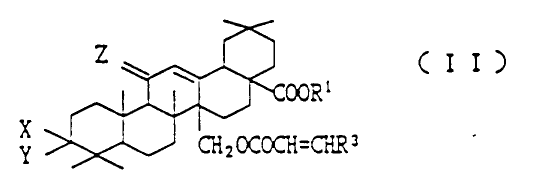 wobei R¹ eine Wasserstoffatom oder eine metabolisierbare Estergruppe ist; R³ ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, oder die Salze davon, umfassend das Kombinieren des Triterpens mit einem pharmazeutisch verträglichen Träger.

5. Verfahren nach Anspruch 4, wobei R¹ ein Wasserstoffatom ist und X und Y zusammen eine Oxogruppe bilden.

6. Verfahren nach Anspruch 4 oder 5, wobei das hergestellte Arzneimittel antagonistische Wirkungen gegenüber Endothelin-Rezeptor aufweist.

7. Verfahren nach Anspruch 4 oder 5, wobei das hergestellte Arzneimittel bei der Prophylaxe oder Behandlung von Krankheiten, die durch Endothelin hervorgerufen werden, verwendbar ist.

## Patentansprüche (Patentansprüche für folgende(n) Vertragsstaat(en): GR)

1. Triterpen der folgenden Formel (I) 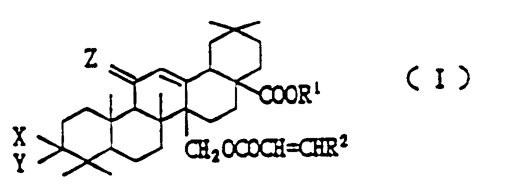 wobei R¹ ein Wasserstoffatom oder eine metabolisierbare Estergruppe ist; R² ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, mit der Maßgabe, daß R² keine 3,4-Dihydroxyphenylgruppe ist, wenn R¹ ein Wasserstoffatom ist und Y eine Hydroxylgruppe ist und weiterhin mit der Maßgabe, daß R² keine 4-Hydroxyphenylgruppe ist, wenn R¹ ein Wasserstoffatom ist, Y eine Hydroxylgruppe ist und Z zwei Wasserstoffatome bedeutet; oder die Salze davon.

2. Verbindung nach Anspruch 1, wobei R¹ ein Wasserstoffatom ist und X und Y zusammen eine Oxogruppe bilden.

3. Verbindung nach Anspruch 1 oder 2, wobei R² eine Phenol-, Hydroxyphenyl- oder Dihydroxyphenylgruppe ist.

4. Verfahren zur Herstellung eines Arzneimittels, umfassend ein Triterpen der folgenden Formel (II) 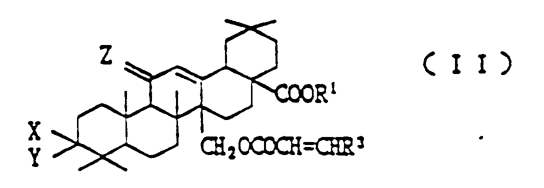 wobei R¹ ein Wasserstoffatom oder eine metabolisierbare Estergruppe ist; R³ ein gegebenenfalls substituierter Arylrest oder ein gegebenenfalls substituierter aromatischer heterocyclischer Rest ist; X ein Wasserstoffatom ist und Y eine Hydroxylgruppe ist oder X und Y zusammen eine Oxogruppe bilden können; Z ein Sauerstoffatom oder zwei Wasserstoffatome bedeutet, oder die Salze davon, umfassend das Kombinieren des Triterpens mit einem pharmazeutisch verträglichen Träger.

5. Verfahren nach Anspruch 4, wobei R¹ ein Wasserstoffatom ist und X und Y zusammen eine Oxogruppe bilden.

6. Verfahren nach Anspruch 4 oder 5, wobei das hergestellte Arzneimittel antagonistische Wirkungen gegenüber Endothelin-Rezeptoren aufweist.

7. Verfahren nach Anspruch 4 oder 5, wobei das hergestellte Arzneimittel bei der Prophylaxe oder Behandlung von Krankheiten, die durch Endothelin hervorgerufen werden, verwendbar ist.

8. Triterpen der folgenden Formel:

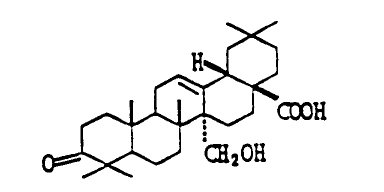

## Revendications (Revendications pour l'(les) Etat(s) contractant(s) suivant(s): AT, BE, CH, DE, DK, FR, GB, IT, LI, LU, MC, NL, SE)

1. Triterpène de la formule (I) suivante : 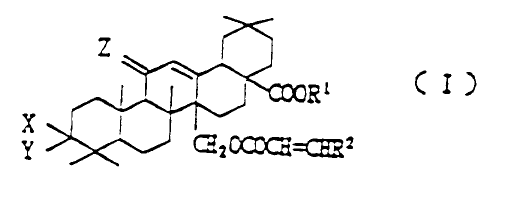 dans laquelle R¹ est de l'hydrogène ou un résidu d'ester métabolisable, R² est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, pour autant que R² ne représente pas un groupe 3,4-dihydroxyphényle lorsque R¹ est de l'hydrogène et Y est de l'hydroxy et de plus pour autant que R² ne représente pas un groupe 4-hydroxyphényle lorsque R¹ est de l'hydrogène, Y est de l'hydroxy et Z représente deux atomes d'hydrogène, ou les sels de celui-ci.

2. Composé suivant la revendication 1, dans lequel R¹ est de l'hydrogène et X et Y forment ensemble un groupe oxo.

3. Composé suivant l'une ou l'autre des revendications 1 et 2, dans lequel R² est un groupe phényle, hydroxyphényle ou dihydroxyphényle.

4. Composition pharmaceutique contenant un triterpène de la formule (II) suivante : 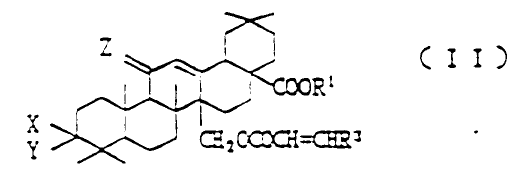 dans laquelle R¹ est un hydrogène ou un résidu d'ester métabolisable, R³ est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, ou les sels de celui-ci.

5. Composition pharmaceutique suivant la revendication 4, dans laquelle R¹ est de l'hydrogène et X et Y forment ensemble un groupe oxo.

6. Composition pharmaceutique suivant l'une ou l'autre des revendications 4 et 5, qui a des activités antagonistes aux récepteurs d'endothéline.

7. Composition pharmaceutique suivant l'une ou l'autre des revendications 4 et 5, utilisable dans la prévention ou le traitement de maladies provoquées par l'endothéline.

8. Triterpène de la formule suivante :

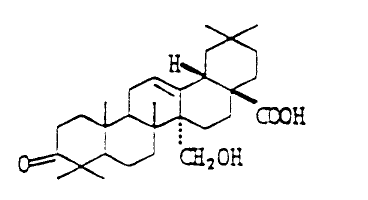

## Revendications (Revendications pour l'(les) Etat(s) contractant(s) suivant(s): ES)

1. Procédé de préparation d'un triterpène de la formule (I) : 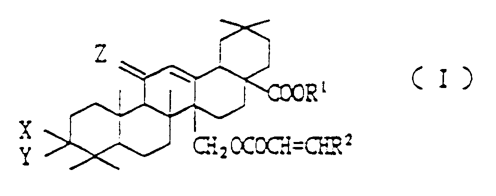 dans laquelle R¹ est de l'hydrogène ou un résidu d'ester métabolisable, R² est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, pour autant que R² ne représente pas un groupe 3,4-dihydroxyphényle lorsque R¹ est de l'hydrogène et Y est de l'hydroxy, ou les sels de celui-ci, qui comprend l'hydrolyse d'un composé de la formule : 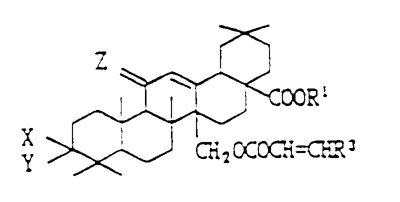 dans laquelle X est de l'hydrogène et Y est de l'hydroxy ou X et Y peuvent former ensemble un groupe oxo, pour donner l'alcool correspondant, qui est ensuite soumis à :
(a) une réaction avec un halogénure d'acide ou un anhydride d'acide des formules :
R²CH=CHCO-halogène ou (R²CH=CHCO)₂O
dans lesquelles R² est tel que défini précédemment, ou
(b) une réaction avec de l'anhydride diméthylphosphonoacétique pour donner le diméthyl phosphonoacétate correspondant, que l'on fait réagir avec un composé de la formule :
R²CHO
dans laquelle R² est tel que défini précédemment,
et, en fonction des nécessités, d'une oxydation ultérieure, suivie d'une éventuelle estérification/formation de sel.

2. Procédé suivant la revendication 1, dans lequel R¹ est de l'hydrogène et X et Y forment ensemble un groupe oxo.

3. Procédé suivant l'une ou l'autre des revendications 1 et 2, dans lequel R² est un groupe phényle, hydroxyphényle ou dihydoxy-phényle.

4. Procédé de préparation d'une composition pharmaceutique contenant un triterpène de la formule (II) suivante : 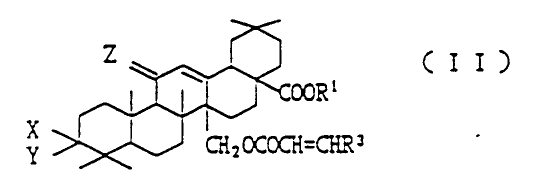 dans laquelle R¹ est de l'hydrogène ou un résidu d'ester métabolisable, R³ est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, ou les sels de celui-ci, comprenant la combinaison de ce triterpène avec un support pharmaceutiquement acceptable.

5. Procédé suivant la revendication 4, dans lequel R¹ est de l'hydrogène et X et Y forment ensemble un groupe oxo.

6. Procédé suivant l'une ou l'autre des revendications 4 et 5, dans lequel la composition pharmaceutique préparée a des activités antagonistes vis-à-vis des récepteurs d'endothéline.

7. Procédé suivant l'une ou l'autre des revendications 4 et 5, dans lequel la composition pharmaceutique préparée est intéressante pour empêcher ou traiter les maladies provoquées par l'endothéline.

## Revendications (Revendications pour l'(les) Etat(s) contractant(s) suivant(s): GR)

1. Triterpène de la formule (I) suivante : 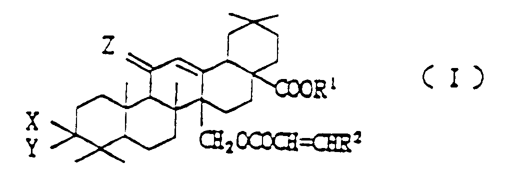 dans laquelle R¹ est de l'hydrogène ou un résidu d'ester métabolisable, R² est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, pour autant que R² ne représente pas un groupe 3,4-dihydroxyphényle lorsque R¹ est de l'hydrogène et Y est de l'hydroxy et de plus pour autant que R² ne représente pas un groupe 4-hydroxyphényle lorsque R¹ est de l'hydrogène, Y est de l'hydroxy et Z représente deux atomes d'hydrogène, ou les sels de celui-ci.

2. Composé suivant la revendication 1, dans lequel R¹ est de l'hydrogène et X et Y forment ensemble un groupe oxo.

3. Composé suivant l'une ou l'autre des revendications 1 et 2, dans lequel R² est un groupe phényle, hydroxyphényle ou dihydroxyphényle.

4. Procédé de préparation d'une composition pharmaceutique contenant un triterpène de la formule (II) suivante : 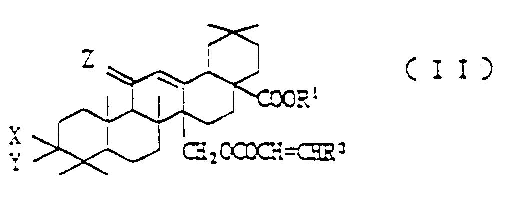 dans laquelle R¹ est de l'hydrogène ou un résidu d'ester métabolisable, R³ est un groupe aryle éventuellement substitué ou un hétérocycle aromatique éventuellement substitué, X est de l'hydrogène et Y est de l'hydroxy ou bien X et Y peuvent former ensemble un groupe oxo, Z est un atome d'oxygène ou deux atomes d'hydrogène, ou les sels de celui-ci, comprenant la combinaison de ce triterpène avec un support pharmaceutiquement acceptable.

5. Procédé suivant la revendication 4, dans lequel R¹ est de l'hydrogène et X et Y forment ensemble un groupe oxo.

6. Procédé suivant l'une ou l'autre des revendications 4 et 5, dans lequel la composition pharmaceutique préparée a des activités antagonistes vis-à-vis des récepteurs d'endothéline.

7. Procédé suivant l'une ou l'autre des revendications 4 et 5, dans lequel la composition pharmaceutique préparée est intéressante pour empêcher ou traiter les maladies provoquées par l'endothéline.

8. Triterpène de la formule suivante :
